# EUROPEAN PATENT SPECIFICATION

(11) **EP 4 192 406 B1**
(45) Date of publication and mention of the grant of the patent: **30.04.2025**
(21) Application number: 20952628.4
(22) Date of filing: 11.11.2020
(51) Int. Cl.: A61F 13/00, C01B 32/00

(54) **PRODUCTION OF A BIODEGRADABLE WOUND DRESSING COMPRISING GRAPHENE-BASED 2,3 DIALDEHYDE BACTERIAL CELLULOSE**
HERSTELLUNG EINES BIOLOGISCH ABBAUBAREN WUNDVERBANDS MIT GRAPHENBASIERTER 2,3-DIALDEHYDBAKTERIENCELLULOSE
PRODUCTION D'UN PANSEMENT BIODÉGRADABLE COMPRENANT DE LA 2,3-DIALDÉHYDE CELLULOSE BACTÉRIENNE À BASE DE GRAPHÈNE

(30) Priority: 03.09.2020 TR 202013979
(43) Date of publication of application: 14.06.2023
(73) Proprietor: Yildiz Teknik Universitesi, 34220 Esenler/ Istanbul (TR); Yildiz Teknoloji Transfer Ofisi Anonim Sirketi, Esenler/Istanbul (TR)
(72) Inventor: SAHIN, Yücel, Esenler / Istanbul (TR); TÜRKOGLU, Nelisa, Besiktas / Istanbul (TR); GÜRSU, Hürmüs, Esenler / Istanbul (TR)
(74) Representative: Yuce, Serfinaz Sibel
(86) International application number: PCT/TR2020/051079
(87) International publication number: WO 2022/050914

(56) References cited:
- US-A1- 2017 151 447
- LI J ET AL: "Preparation and characterization of 2,3-dialdehyde bacterial cellulose for potential biodegradable tissue engineering scaffolds", MATERIALS SCIENCE AND ENGINEERING C, ELSEVIER SCIENCE S.A, CH, vol. 29, no. 5, 1 June 2009 (2009-06-01), pages 1635 - 1642, XP026107130, ISSN: 0928-4931, [retrieved on 20090120], DOI: 10.1016/J.MSEC.2009.01.006
- PORTELA RAQUEL ET AL: "Bacterial cellulose: a versatile biopolymer for wound dressing applications", MICROBIAL BIOTECHNOLOGY, vol. 12, no. 4, 5 March 2019 (2019-03-05), GB, pages 586 - 610, XP055843182, ISSN: 1751-7915, Retrieved from the Internet <URL:https://onlinelibrary.wiley.com/doi/full-xml/10.1111/1751-7915.13392> [retrieved on 20231116], DOI: 10.1111/1751-7915.13392
- MOHAMMADNEJAD JAVAD ET AL: "Graphene oxide/silver nanohybrid: Optimization, antibacterial activity and its impregnation on bacterial cellulose as a potential wound dressing based on GO-Ag nanocomposite-coated BC", ENGINEERING IN LIFE SCIENCES, vol. 18, no. 5, 1 May 2018 (2018-05-01), DE, pages 298 - 307, XP055913467, ISSN: 1618-0240, Retrieved from the Internet <URL:https://www.ncbi.nlm.nih.gov/pmc/articles/PMC6999449/pdf/ELSC-18-298.pdf> [retrieved on 20231116], DOI: 10.1002/elsc.201700138
- YAO MINGJUN ET AL: "Preparation of dialdehyde cellulose graftead graphene oxide composite and its adsorption behavior for heavy metals from aqueous solution", CARBOHYDRATE POLYMERS, vol. 212, 18 February 2019 (2019-02-18), pages 345 - 351, XP085615359, ISSN: 0144-8617, DOI: 10.1016/J.CARBPOL.2019.02.052
- MOHAMMADNEJAD, J ET AL.: "Graphene oxide/silver nanohybrid: Optimization, antibacterial activity and its impregnation on bacterial cellulose as a potential wound dressing based on GO- Ag nanocomposite-coated BC", ENG. LIFE SCI., vol. 18, 2018, pages 298 - 307, XP055913467, DOI: https://doi.org/10.1002/ elsc.201700138
- JIAN LI ET AL.: "Preparation and characterization of 2,3-dialdehyde bacterial cellulose for potential biodegradable tissue engineering scaffolds", MATERIALS SCIENCE AND ENGINEERING: C, vol. 29, no. 5, 2009, pages 1635 - 1642, XP026107130, ISSN: 0928-4931, DOI: https://doi.org/10.1016/j.msec. 2009.01.00 6
- RAQUEL PORTELA CATARINA R. ET AL.: "Bacterial cellulose: a versatile biopolymer for wound dressing applications", MICROBIAL BIOTECHNOLOGY, vol. 12, no. 4, 2019, pages 586 - 610, XP055843182, DOI: 10.1111/1751-7915.13392

## Description

### Technical Field of the Invention

The present invention relates to wound dressings used for the treatment of wounds defined as open wounds when they become chronic in the health sector.

The present invention more specifically relates to a wound dressing material and a production method thereof, wherein the inventive wound dressing material comprises graphene-based 2,3 dialdehyde bacterial cellulose compound and is capable of supporting the cells in the wound area to heal chronic, i.e. non-healing wounds (e.g. bedsores), eliminating the risk of infection development in an open wound.

### State of the Art

The world of medicine frequently encounters wounds that are defined as open wounds when these wounds do not heal and become chronic. Wounds such as these, i.e. bedsores (pressure ulcers) or wounds stemming from diabetes, or non-healing wounds stemming from circulatory disorders severely reduce the patients' quality of life. Conventional wound dressing materials are used in order to support the healing process of such wounds. Conventionally, materials used for wound care and treatment include materials such as natural and synthetic bandages, hydrophilic cotton, roll bandages, and medical gauzes. The reason underlying the use of such wound dressings is to allow the wound exudate to vaporize, thereby allowing the wound to dry and preventing bacterial growth in the wound medium. However, these conventional approaches fail to provide the optimal environment and conditions necessary for tissue regeneration. Nowadays, it is known for a fact that maintaining the warm and wet environment created around the wound is necessary for the regeneration of the tissue in the wound area. Therefore, nowadays materials that allow the movement of epithelial cells during the healing process of the wound, enable the oxygen circulation therein, prevent bacterial contamination, and allow for creating optimal ambiance conditions are being developed and used. In more advanced nanotechnological materials, however, it is a much more preferred approach where the material is absorbed by the body after the epithelial cell proliferation has reached a certain level while enabling the movement of epithelial cells during the healing process as mentioned above. Such modern materials provide a much faster and successful wound healing process. A frequently implemented method for providing antimicrobial properties to wound dressing materials involves adding silver nanoparticles either to wound dressing material or to the structure of the material.

There is a number of commercially available wound dressings comprising various raw materials. While some of these feature a hydro-colloidal structure, some have a hydrogel structure. This particular difference stems from the fact that these two different materials of different properties (i.e. hydro-colloidal and hydrogel) are specific to two different wound types (i.e. dry or exudated). Generally, hydrogels are used either as elastic films (sheets) or amorphous gels. Nowadays, hydrogels used in the form of films have many of the optimal wound dressing properties. Moreover, these film-shaped hydrogels in the form of thin layers may be cut and prepared in conformity with the shape of the wound due to their convenient structure. When hydrogel dressings are applied to a dry wound surface, they moisturize the wound, thereby creating the ideal wet environment required for the healing of the wound. Another advantage of these hydrogels is that they may easily be shaped and cleaned from the surface of the wound. Wound dressing materials with hydrogel properties may be obtained from a number of natural or synthetic polymers with hydrophilic and water-swelling properties such as cross-linked carboxymethyl cellulose, modified starch, alginate, or pectin. However, many of the commercially available hydrogel wound dressings are made of synthetic polymers. Needless to say, there are also a considerable number of wound dressing materials produced by using natural polymers. However, these materials prepared by using such natural polymers are subjected to various chemical processes in order to create a hydrogel membrane. Removal of the used chemicals from the prepared film layer subsequent to said chemical processes requires an additional process step, and of course, brings along additional costs. It is known for a fact that undesired chemical residue may induce cytotoxic, allergic, or even mutagenic effects in cells located in the area of application.

The list provided below shows the respective literature publications, which have been determined as a result of the search conducted and which form the state of the art, on bacterial cellulose wound dressing materials and graphene.

Azarniya, A., Eslahi, N., Mahmoudi, N., & Simchi, A. (2016). Effect of graphene oxide nanosheets on the physicomechanical properties of chitosan/bacterial cellulose nanofibrous composites. Composites Part A: Applied Science and Manufacturing, 85, 113-122.

Czaja, W., Krystynowicz, A., Bielecki, S., & Brown Jr, R. M. (2006). Microbial cellulose-the natural power to heal wounds. Biomaterials, 27(2), 145-151.

Chook, S. W., Chia, C. H., Zakaria, S., Ayob, M. K., Huang, N. M., Neoh, H. M., & Jamal, R. (2015). Antibacterial hybrid cellulose-graphene oxide nanocomposite immobilized with silver nanoparticles. RSC Advances, 5(33), 26263-26268.

de Moraes, A. C. M., Andrade, P. F., de Faria, A. F., Simões, M. B., Salomão, F. C. C. S., Barros, E. B., & Alves, O. L. (2015). Fabrication of transparent and ultraviolet shielding composite films based on graphene oxide and cellulose acetate. Carbohydrate polymers, 123, 217-227.

Fan, Z., Liu, B., Wang, J., Zhang, S., Lin, Q., Gong, P., ... & Yang, S. (2014). A novel wound dressing based on Ag/graphene polymer hydrogel: effectively kill bacteria and accelerate wound healing. Advanced Functional Materials, 24(25), 3933-3943.

Gürsu, H., Gencten, M., & Sahin, Y. (2017). One-step electrochemical preparation of graphene-coated pencil graphite electrodes by cyclic voltammetry and their application in vanadium redox batteries. Electrochimica Acta, 243, 239-249.

Gürsu, H., Gençten, M., & Sahin, Y. (2018). Preparation of N-doped graphene-based electrode via the electrochemical method and its application in vanadium redox flow battery. International Journal of Energy Research, 42(12), 3851-3860.

Hakkarainen, T., Koivuniemi, R., Kosonen, M., Escobedo-Lucea, C., Sanz-Garcia, A., Vuola, J., ... & Yliperttula, M. (2016). Nanofibrillar cellulose wound dressing in skin graft donor site treatment. Journal of controlled release, 244, 292-301.

Hoon, R., Oster, G. A., Damien, C., Wang, J., & Serafica, G. (2005). U.S. Patent Application No. 10/864,804.

Kaplan, E., Ince, T., Yorulmaz, E., Yener, F., Harputlu, E., & Laçin, N. T. (2014). Controlled delivery of ampicillin and gentamycin from cellulose hydrogels and their antibacterial efficiency. Journal of Biomaterials and Tissue Engineering, 4(7), 543-549.

Laçin, N. T. (2014). "Development of biodegradable antibacterial cellulose-based hydrogel membranes for wound healing." International journal of biological macromolecules 67: 22-27. Li,J. et al., "Preparation and characterization of 2,3-dialdehyde bacterial cellulose for potential biodegradable tissue engineering scaffolds". Material science and engineering, 29(5), 1635-1642

Lin, W. C., Lien, C. C., Yeh, H. J., Yu, C. M., & Hsu, S. H. (2013). Bacterial cellulose and bacterial cellulose-chitosan membranes for wound dressing applications. Carbohydrate polymers, 94(1), 603-611.

Luo, H., Ao, H., Li, G., Li, W., Xiong, G., Zhu, Y., & Wan, Y. (2017). Bacterial cellulose/graphene oxide nanocomposite as a novel drug delivery system. Current Applied Physics, 17(2), 249-254.

Luo, H., Xie, J., Wang, J., Yao, F., Yang, Z., & Wan, Y. (2018). Step-by-step self-assembly of 2D few-layer reduced graphene oxide into the 3D architecture of bacterial cellulose for a robust, ultralight, and recyclable all-carbon absorbent. Carbon, 139, 824-832.

Maneerung, T., Tokura, S., & Rujiravanit, R. (2008). Impregnation of silver nanoparticles into bacterial cellulose for antimicrobial wound dressing. Carbohydrate polymers, 72(1), 43-51.

Picheth, G. F., Pirich, C. L., Sierakowski, M. R., Woehl, M. A., Sakakibara, C. N., de Souza, C. F., ... & de Freitas, R. A. (2017). Bacterial cellulose in biomedical applications: a review. International journal of biological macromolecules, 104, 97-106.

Shaw, H., & Linnane, P. G. (2010). U.S. Patent No. 7,759,539. Washington, DC: U.S. Patent and Trademark Office.

Shao, W., Liu, H., Liu, X., Wang, S., & Zhang, R. (2015). Antibacterial performances and biocompatibility of bacterial cellulose/graphene oxide composites. RSC Advances, 5(7), 4795-4803.

Solway, D. R., Consalter, M., & Levinson, D. J. (2010). Microbial cellulose wound dressing in the treatment of skin tears in the frail elderly. Wounds, 22(1), 17.

Sahin Y, Hürmüs GÜRSU, Metin GENÇTEN, "A method in order to generate graphene-based electrode", TURKEY, Patent, PCT/TR2017/050121, October 2017

Unnithan, A. R., Gnanasekaran, G., Sathishkumar, Y., Lee, Y. S., & Kim, C. S. (2014). Electrospun antibacterial polyurethane-cellulose acetate-zein composite mats for wound dressing. Carbohydrate polymers, 102, 884-892.

Wu, J., Zheng, Y., Song, W., Luan, J., Wen, X., Wu, Z., ... & Guo, S. (2014). In situ synthesis of silvernanoparticles/bacterial cellulose composites for slow-released antimicrobial wound dressing. Carbohydrate polymers, 102, 762-771.

Zhu, W., Li, W., He, Y., & Duan, T. (2015). In-situ biopreparation of biocompatible bacterial cellulose/graphene oxide composites pellets. Applied Surface Science, 338, 22-26.

Neither the domestic nor the foreign publications disclose a wound dressing material comprising both bacterial cellulose and graphene in the structure thereof as in the present invention.

Furthermore, the respective research conducted in this regard showed no similar commercialized product available in the market. There are both domestic and foreign scientific publications available -as stated above- in which various cellulose wound dressings are disclosed. However, since there is no wound dressing that comprises both materials together, the inventive wound dressing provides novelty by this technical element. In addition thereto, a production method has been developed for the production of such wound dressing materials.

Furthermore, the inventors have discovered that the inventive wound dressing material produced by means of the inventive production method supports the cells in the wound area for the healing process of chronic, i.e. non-healing wounds and eliminates the risk of infection development in an open wound.

### Description of the Invention

The object of the present invention is to provide a graphene-based 2,3 dialdehyde bacterial cellulose wound dressing material and a production method thereof, that is capable of supporting the cells in the wound area for the care and treatment of chronic, i.e. non-healing wounds (e.g. bedsores), and of eliminating the risk of infection development in an open wound. The inventive graphene-based cellulose wound dressing is a dressing that comprises hydrogel and graphene derivatives of film type having 2,3 dialdehyde bacterial cellulose structure, and antimicrobial effects. Graphene-based materials need to provide antibacterial and physical properties to a wound dressing. The present invention stimulates the healing of chronic, i.e. non-healing wounds (e.g. bedsores).

Graphene derivatives to be used in the inventive wound dressing may be one or more materials selected from; graphene and graphene materials doped with -N, -S, -Cl, -P, -B, -F, -Si, - Ti; graphene oxide and graphene oxide materials doped with - N, -S, -Cl, -P, -B, -Si, -F; graphene derivatives with functionalized -Si, -B₂O₃ -NH₂, -ClO₃, -B₂O₃, -P₂O₅ groups. More preferably, graphene derivatives synthesized with Yucel's Method as disclosed in the respective literature.

### Description of the Figures

- **FIGURE 1**: illustrates the (a) AFM and (b) SEM views of bacterial cellulose provided with biodegradability property (Lacin 2014).
- **FIGURE 2**: illustrates the cell viability results of various synthesized graphene derivatives.
- **FIGURE 3**: illustrates the effects of graphene derivatives on IL-1β synthesis on macrophages.

### Detailed Description of the Invention

The present invention relates to a wound dressing material developed for wound dressings that support the cells in the wound area for the treatment of wounds and eliminates the risk of infection development in open wounds, wherein the present invention discloses a wound dressing material comprising graphene-based 2,3 dialdehyde bacterial cellulose.

The graphene material to be used herein may be one or more materials selected from; graphene and graphene materials doped with -N, -S, -Cl, -P, -B, -F, -Si, -Ti; graphene oxide and graphene oxide materials doped with -N, -S, -Cl, -P, -B, -Si, -F or graphene derivatives with functionalized -Si, -B₂O₃ -NH₂, -ClO₃, -B₂O₃, -P₂O₅ groups.

Another novel property of the present invention is that it comprises a method for preparing graphene-loaded 2,3 dialdehyde bacterial cellulose wound dressing material for wound dressings that supports cells in the wound area for the treatment of chronic wounds and that eliminates the risk of infection development in open wounds. In general, the inventive method for preparing wound dressing material comprising graphene-loaded 2,3 dialdehyde bacterial cellulose comprises the following process steps:
- Producing the bacterial cellulose and rendering it biodegradable (forming 2,3 dialdehyde groups),
- Synthesizing graphene or graphene derivatives,
- Loading the obtained bacterial cellulose membrane with graphene and graphene derivatives in a range between 1% and 15% by weight,

As mentioned in the preparation method, different graphene or graphene derivatives may be one or more materials selected from; graphene and graphene materials doped with -N, -S, -Cl, -P, -B, -F, -Si, -Ti; graphene oxide and graphene oxide materials doped with -N, -S, -Cl, -P, -B, -Si, -F or graphene derivatives with functionalized -Si, B₂O₃, -NH₂, -ClO₃, -B₂O₃, -P₂O₅ groups.

A method implemented in previous studies of inventors is used in the production stage of the bacterial cellulose as provided in the preparation method.

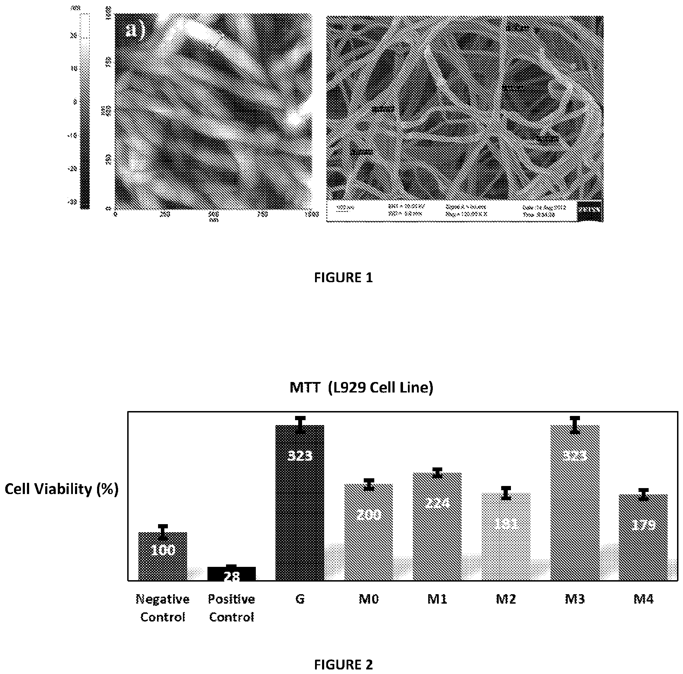

Cellulose is a biopolymer naturally available in large quantities in plant structures. As cellulose may be obtained from a vegetative source, it may also be produced by bacteria. The bacterial cellulose (BC) used in the present invention is produced via a previously implemented method. Two different media are prepared for the production of the bacterial cellulose. One of the two media is a medium that will be prepared for inoculum formation. It is prepared so as to contain 25 g/L of the medium, 3 g/L of Peptone, and 5 g/L of yeast extract, the pH value thereof is adjusted to 5.0 via 1 M hydrochloric acid (HCl) and sterilized by autoclaving for 20 minutes at 120°C. 25 mL of the medium is transferred to Erlenmeyer flasks of 100mL, and Acetobacter xylinum (ATCC 10245) bacteria are added thereto. The obtained media are agitated at 30°C and 150 rpm for a duration of 2-3 days, thereby obtaining the inoculum. The medium necessary for the production of cellulose for inoculums is prepared so as to contain 20 g/L of glucose, 10 g/L of Peptone, 10 g/L of yeast extract, 8mM of KH₂PO₄, and 12mM of K₂HPO₄, the pH value thereof is adjusted to 5.0 via 1 M hydrochloric acid (HCl) and sterilized by autoclaving for 20 minutes at 120°C. Inoculum is added to the medium being divided into glass containers, at a ratio of 1/10, and left for incubation at 30°C for a duration of 7 days. Membranes are kept inside 1 M of NaOH in a water bath adjusted to 80°C for 2 hours in order to purify the cellulose membranes formed on the surface of the glass from bacterial and cellular impurities. Subsequently, they are washed with distilled water until the pH value thereof reaches 7.0. Prepared membranes are autoclaved and preserved. The production of bacterial cellulose was optimized as a result of previous studies conducted by our group (Lacin 2014). Figure 1A illustrates the AFM and SEM photos of bacterial cellulose. Bacterial cellulose was rendered biodegradable in previous studies conducted by our group (Lacin 2014).

The second stage of the inventive method for preparing wound dressing material comprising graphene-loaded 2,3 dialdehyde bacterial cellulose in which graphene derivatives are synthesized and characterized via Yucel's Method is performed as stated below.

Synthesis of graphene-based materials is performed via a method called Yucel's Method (H. Gürsu vd. 2019) which is a method developed by the team of inventors. Graphene and derivatives thereof (graphene derivatives comprising graphene and graphene doped with -N, -S, -Cl, -P, -B, -F, -Si and functional groups such as -NO₃, -NH₂, -ClO₃, -B₂O₃, -P₂O₅) are synthesized by our group by using the all of the aforementioned methods.

A conventional triple electrode system is used for preparing graphene-based materials from graphite-based materials via the electrochemical Yucel's Method. Ag/AgCl, calomel, Hg/HgSO₄ are used as the reference electrode, while Pt is used as a counter electrode and a graphite-based electrode is used as the working electrode. Solutions comprising salts, monoprotic, polyprotic acids, bases and mixtures thereof containing various functional groups in their structures are used as the electrolyte within a concentration range between 0.0001 M - 10 M. Unlike the literature, voltage ratings being worked with for graphene production via electrochemical method are low and requires applying less energy. The synthesis method is implemented between (-3 V) - (+6 V) working potential range; in a scanning rate value between 0.001 V/s - 1V/s and in a cycle changing between 1 - 500.

In summary, the second stage of the inventive method for preparing wound dressing material comprising graphene-loaded 2,3 dialdehyde bacterial cellulose in which graphene derivatives are synthesized via Yucel's Method comprises the following process steps;
- Preparing solutions comprising salts, monoprotic, polyprotic acids, bases, and mixtures thereof containing various functional groups in their structures as the electrolyte within a concentration range between 0.0001 M - 10 M;
- Using Ag/AgCl, calomel, Hg/HgSO₄ as the reference electrode, Pt as the counter electrode, and graphite-based electrode as the working electrode;
- Performing graphene synthesis such that it is between (-3 V) - (+6 V) working potential range; in a scanning rate value between 0.001 V/s - 1V/s and a cycle changing between 1 - 500.

The third stage of the inventive method for preparing wound dressing material comprising graphene-loaded 2,3 dialdehyde bacterial cellulose comprises preparing and characterizing graphene-doped wound dressing materials.

Studies conducted in the field of tissue engineering have proven that bacterial cellulose is the ideal material for 3D printing with nanofibrils. Being able to control the mechanical properties of bacterial cellulose materials is among its most important advantages. In this context, preparation and analysis of cellulose nanofibrils for 3D bioprinters were reported by Torres-Rendon et al. The work of Torres-Rendon et al. demonstrated that the obtained hydrogels may be processed, printed in different shapes and structures obtained after the printing processes are rehydrated (Torres-Rendon vd. 2016).

The preliminary studies have concluded that graphene types affect cell growth positively.

A graphite rod as the working electrode, to be used in the tests mentioned above, was obtained via Yucel's Method, inside nitric acid solutions such that it has a potential between 1.0 and +2.3 V, between -0.7 and +2.3 V, and between 0 and +2.3 V, and inside the Ag/AgCl (at 3M KCl) mixture at room temperature (25 °C) in 100 voltammetric cycles (with a scanning rate of 50mV/s) to produce approximately 1.0 g of graphene powder. Graphene-based materials are named as; graphite (G), N-doped graphene oxide (M0), N-doped graphene1 (M1), N-doped graphene2 (M2), N-doped graphene3 (M3), and N-doped graphene4 (M4).

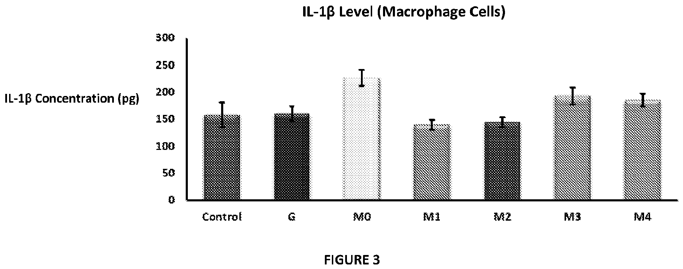

Cell viability and immunology assays were performed on the N-doped graphene- and graphene oxide-based materials and the results obtained are provided above. In this context, (3-(4,5-Dimethylthiazol-2-yl)-2,5-Diphenyltetrazolium Bromide (MTT) assay, which is a colorimetric method, was performed on the L929 cell line for the cell viability assay. Graphene samples at a concentration of 0.2g/mL were used for MTT and IL-1 β assays. Respective test results indicated that all graphene groups induced cell proliferation. Figure 2 illustrates the MTT assay results of graphene (G) and graphene derivatives (M0, M1, M2, M3, and M4) comprising various functional groups. Cell viabilities were calculated as; 323% in the G group, 200% in M0, 224% in M1, 181% in M2, 323% in M3, and 179% in the M4 group. All graphene groups subjected to tests have been determined to induce cellular division. IL-1β levels were determined by using human macrophage cells to be able to foresee graphene groups' immune response in the body and it was observed that apart from the M0 group, none of the graphene groups created a significant difference in the IL-1β amount secreted by the cells. Macrophage cells used at this stage were obtained by stimulating the THP-1 (monocyte) cell line. IL-1β levels were measured by using a commercial ELISA kit. Considering the results (Figure 3) obtained, it was determined that it would not produce a significant immune response.

Bacterial cellulose and graphene derivatives were separately produced by our group. Bacterial cellulose solutions comprising graphene in a range between 1% and 15% by weight (with heteroatom) and other graphene derivative concentrations will be prepared in preparation of graphene-doped bacterial cellulose wound dressings.

The inventive graphene-based bacterial cellulose wound dressing appeals to a large market volume in the health sector. The production of bacterial cellulose is already being carried out on an industrial scale. Wound dressing materials with graphene content will be produced on an industrial scale by implementing the inventive method developed as an alternative to the industrial production method utilized at present.

## Claims

1. A wound dressing material that supports cells in the wound area for the treatment of chronic wounds and that eliminates the risk of infection development in open wounds, **characterized in that;** it comprises graphene-based 2,3 dialdehyde bacterial cellulose compound.

2. Wound dressing material according to Claim 1, **characterized in that;** the graphene material is a material selected from one or more of; graphene and graphene materials doped with -N, -S, -Cl, -P, -B, -F, -Si, -Ti; graphene oxide and graphene oxide materials doped with -N, -S, -Cl, -P, -B, -Si, -F or graphene derivatives with functionalized -Si, -B₂O₃ -NH₂, -ClO₃, -B₂O₃, -P₂O₅ groups.

3. A method for preparing wound dressing material according to Claim 1, **characterized in that,** it comprises the process steps of;
- Producing the bacterial cellulose and rendering it biodegradable (forming 2,3 dialdehyde groups),
- Synthesizing graphene or graphene derivatives,
- Loading the obtained bacterial cellulose membrane with graphene and graphene derivatives in a range between 1% and 15% by weight.

4. A method for preparing wound dressing material according to Claim 3, **characterized in that,** it comprises the process steps of;
- preparing solutions comprising salts, monoprotic, polyprotic acids, bases and mixtures thereof containing various functional groups in their structures as the electrolyte within a concentration range between 0.0001 M - 10 M;
- Using Ag/AgCl, calomel, Hg/HgSO₄ as the reference electrode, Pt as the counter electrode, and graphite-based electrode as the working electrode;
- Performing graphene synthesis such that it is between (-3 V) - (+6 V) working potential range; in a scanning rate value between 0.001 V/s - 1V/s and a cycle changing between 1 - 500.

5. A method for preparing wound dressing material according to Claim 3, **characterized in that,** it comprises the process step of selecting various graphene or graphene derivatives from one or more of; graphene and graphene materials doped with N, S, Cl, P, B, F, Si, Ti; graphene oxide and graphene oxide materials doped with -N, -S, - Cl, -P, -B, -Si, -F or graphene derivatives with functionalized -Si, -B₂O₃, -NH₂, -ClO₃, -B₂O₃, -P₂O₅ groups.

6. A method for preparing wound dressing material according to Claim 3, **characterized in that,** it comprises the process steps of;
- Preparing two different media for the production of the bacterial cellulose,
- Preparing it so as to contain 25 g/L of the medium, 3 g/L of Peptone and 5 g/L of yeast extract, adjusting the pH value thereof to 5.0 via 1 M hydrochloric acid (HCl), and sterilizing it by autoclaving for 20 minutes at 120°C,
- transferring 25 mL of medium to Erlenmeyer flasks of 100mL, and adding Acetobacter xylinum (ATCC 10245) bacteria thereto,
- Agitating the obtained media at 30°C and 150 rpm for a duration of 2-3 days, thereby obtaining the inoculum,
- Preparing the medium necessary for the production of cellulose for inoculums so as to contain 20 g/L of glucose, 10 g/L of Peptone, 10 g/L of yeast extract, 8mM of KH₂PO₄, and 12mM of K₂HPO₄, adjusting the pH value thereof to 5.0 via 1 M hydrochloric acid (HCl) and sterilizing it by autoclaving for 20 minutes at 120°C,
- Adding the inoculum to the medium being divided into glass containers, at a ratio of 1/10 and leaving it for incubation at 30°C for a duration of 7 days,
- Keeping the membranes inside 1 M of NaOH in a water bath adjusted to 80°C for 2 hours in order to purify the cellulose membranes formed on the surface of the glass from bacterial and cellular impurities, and washing them with distilled water until the pH value thereof reaches 7.0, and
- Preserving the prepared membranes by autoclaving.

## Patentansprüche

1. Wundverbandmaterial, das Zellen im Wundbereich zur Behandlung chronischer Wunden unterstützt und das Risiko der Entwicklung einer Infektion in offenen Wunden beseitigt, **dadurch gekennzeichnet, dass** es eine 2,3-Dialdehyd-Bakterienzelluloseverbindung auf Graphenbasis umfasst.

2. Wundverbandmaterial nach Anspruch 1, **dadurch gekennzeichnet, dass** das Graphenmaterial ein Material ist, das aus einem oder mehreren der folgenden ausgewählt ist: Graphen und Graphenmaterialien, die mit -N, -S, -C1, -P, -B, -F, -Si, -Ti; Graphenoxid und Graphenoxidmaterialien, die mit -N, - S, -C1, -P, -B, -Si, -F dotiert sind; oder Graphenderivate mit funktionalisierten -Si, -B203, -NH2, -C103, -B2O5, -P2O5-Gruppen.

3. Verfahren zur Herstellung eines Wundverbandsmaterials nach Anspruch 1, **dadurch gekennzeichnet, dass** es die folgenden Verfahrensschritte umfasst: - Herstellung der bakteriellen Cellulose und deren biologische Abbaubarkeit (Bildung von 2,3-Dialdehydgruppen), - Synthese von Graphen oder Graphenderivaten, - Beladung der erhaltenen bakteriellen Cellulosemembran mit Graphen und Graphenderivaten in einem Bereich zwischen 1 und 15 Gew.-%.

4. Verfahren zur Herstellung von Wundverbandmaterial nach Anspruch 3, **dadurch gekennzeichnet, dass** es die folgenden Verfahrensschritte umfasst:
- Herstellung von Lösungen, die Salze, einwertige und mehrwertige Säuren, Basen und Gemische davon umfassen, die in ihren Strukturen verschiedene funktionelle Gruppen enthalten, als Elektrolyt in einem Konzentrationsbereich zwischen 0,0001 M und 10 M;
- Verwendung von Ag/AgCl, Kalomel, Hg/HgSO₄ als Referenzelektrode, Pt als Gegenelektrode und einer Elektrode auf Graphitbasis als Arbeitselektrode;
- Durchführung der Graphensynthese so, dass sie zwischen dem Arbeitspotentialbereich von (-3 V) (+6 V) liegt; bei einer Abtastrate zwischen 0,001 V/s und 1 V/s und einem Zykluswechsel zwischen 1 und 500.

5. A method Verfahren zur Herstellung eines Wundverbandmaterials nach Anspruch 3, **dadurch gekennzeichnet, dass** es den Schritt des Auswählens verschiedener Graphen oder Graphenderivate aus einem oder mehreren der folgenden Graphen und Graphenmaterialien, die mit N, S, Cl, P, B, F, Si, Ti dotiert sind; Graphenoxid und Graphenoxidmaterialien, die mit -N, -S, Cl, -P, - B, -Si, -F dotiert sind, oder Graphenderivate mit funktionalisierten -Si, -B203, -NH2, -C103, -B2O5, -P2O5-Gruppen.

6. Verfahren zur Herstellung eines Wundverbandsmaterials nach Anspruch 3, **dadurch gekennzeichnet, dass** es die folgenden Verfahrensschritte umfasstof;
- Vorbereitung von zwei verschiedenen Medien für die Produktion der bakteriellen Cellulose,
- Vorbereitung, so dass sie 25 g/l des Mediums, 3 g/l Pepton und 5 g/l Hefeextrakt enthält, Einstellung des pH-Werts auf 5,0 mit 1 M Salzsäure (HC1) und Sterilisierung durch 20-minütiges Autoklavieren bei 120 °C,
- 25 ml Medium in 100-ml-Erlenmeyerkolben umfüllen und Acetobacter-xylinum-Bakterien (ATCC 10245) hinzufügen,
- die erhaltenen Medien bei 30 °C und 150 U/min für eine Dauer von 2-3 Tagen schütteln, wodurch das Inokulum entsteht,
- Vorbereitung des für die Produktion von Zellulose für Inokula erforderlichen Mediums, sodass es 20 g/l Glukose, 10 g/l Pepton, 10 g/l Hefeextrakt, 8 nM KH₂PO₄ und 12 mM K₂HPO₄ enthält, Einstellung des pH-Werts auf 5, 0 mittels 1 M Salzsäure (HC1) eingestellt und durch 20-minütiges Autoklavieren bei 120 °C sterilisiert wird,
- Zugabe des Inokulums zum Medium, das in Glasbehälter aufgeteilt wird, in einem Verhältnis von 1/10 und Inkubation bei 30 °C für eine Dauer von 7 Tagen,
- Die Membranen 2 Stunden lang in 1 M NaOH in einem auf 80 °C eingestellten Wasserbad aufbewahren, um die auf der Glasoberfläche gebildeten Zellulosemembranen von bakteriellen und zellulären Verunreinigungen zu reinigen, und sie mit destilliertem Wasser waschen, bis der pH-Wert 7,0 erreicht, und
- - die vorbereiteten Membranen durch Autoklavieren konservieren.

## Revendications

1. Un matériau de pansement pour plaies, conçu pour soutenir les cellules dans la zone de la plaie lors du traitement des plaies chroniques et pour prévenir le risque de développement d'infections dans les plaies ouvertes, **caractérisé en ce qu'**il comprend un composé de cellulose bactérienne 2,3-dialdéhyde à base de graphène.

2. Matériau de pansement selon la revendication 1, **caractérisé en ce que** le matériau à base de graphène est un matériau sélectionné parmi un ou plusieurs des éléments suivants: le graphène et les matériaux de graphène dopés avec -N, -S, -C1, -P, -B, -F, -Si, -Ti; l'oxyde de graphène et les matériaux d'oxyde de graphène dopés avec -N, -S, -C1, -P, -B, -Si, -F ou des dérivés de graphène fonctionnalisés avec des groupes --Si, -B₂0₃ -NH₂, -C10₃, -B₂O₅, - P₂O₅.

3. Un procédé de préparation d'un matériau de pansement selon la revendication 1, **caractérisé en ce qu'**il comprend les étapes suivantes:
- Produire la cellulose bactérienne et la rendre biodégradable (formation de groupes 2,3-dialdéhyde),
- Synthétiser le graphène ou des dérivés de graphène,
- Charger la membrane de cellulose bactérienne obtenue avec du graphène et des dérivés de graphène dans une proportion comprise entre 1 % et 15 % en poids.

4. Un procédé de préparation d'un matériau de pansement selon la revendication 3, **caractérisé en ce qu'**il comprend les étapes suivantes:
- Préparer des solutions comprenant des sels, des acides monoprotique et polyprotique, des bases et leurs mélanges contenant divers groupes fonctionnels dans leurs structures en tant qu'électrolyte, dans une gamme de concentration comprise entre 0,0001 M et 10 M;
- Utiliser Ag/AgCl, calomel, Hg/HgSO₄ comme électrode de référence, Pt comme électrode auxiliaire et une électrode à base de graphite comme électrode de travail ;
- Réaliser la synthèse du graphène dans une plage de potentiel de travail comprise entre (-3 V) et (+6 V), avec une vitesse de balayage comprise entre 0,001 V/s et 1 V/s et un nombre de cycles variant entre 1 et 500.

5. Un procédé de préparation d'un matériau de pansement selon la revendication 3, **caractérisé en ce qu'**il comprend l'étape de sélection de divers graphènes ou dérivés de graphène parmi un ou plusieurs des éléments suivants: le graphène et les matériaux de graphène dopés avec N, S, C1, P, B, F, Si, Ti; l'oxyde de graphène et les matériaux d'oxyde de graphène dopés avec -N, -S, C1, -P, -B, -Si, -F ou des dérivés de graphène fonctionnalisés avec des groupes -Si, - B₂O₃, -NH₂, -ClO₃, -B₂O₃, -P₂O₅.

6. Un procédé de préparation d'un matériau de pansement selon la revendication 3, **caractérisé en ce qu'**il comprend les étapes suivantes:
- Préparer deux milieux différents pour la production de cellulose bactérienne,
- Préparer un milieu contenant 25 g/L de substrat, 3 g/L de peptone et 5 g/L d'extrait de levure, ajuster son pH à 5,0 à l'aide d'acide chlorhydrique (HC1) 1 M, et le stériliser par autoclavage pendant 20 minutes à 120°C,
- Transférer 25 mL de milieu dans des erlenmeyers de 100 mL et y ajouter la bactérie Acetobacter xylinum (ATCC 10245),
- Incuber le mélange à 30°C avec agitation à 150 rpm pendant 2 à 3 jours pour obtenir l'inoculum ;
- Préparer le milieu de culture pour la production de cellulose en y incorporant 20 g/L de glucose, 10 g/L de peptone, 10 g/L d'extrait de levure, 8 mM de KH₂PO₄ et 12 mM de K₂HPO₄, ajuster son pH à 5,0 avec de l'acide chlorhydrique (HCl) 1 M, et le stériliser par autoclavage pendant 20 minutes à 120°C ;
- Ajouter l'inoculum au milieu divisé en récipients en verre, à un ratio de 1/10, et laisser incuber à 30°C pendant 7 jours,
- Placer les membranes formées à la surface des récipients en verre dans une solution de NaOH 1 M dans un bain-marie à 80°C pendant 2 heures afin de les purifier des impuretés bactériennes et cellulaires, puis les laver à l'eau distillée jusqu'à atteindre un pH de 7.0,
- Conserver les membranes préparées par autoclavage.
